# EUROPÄISCHE PATENTSCHRIFT

(11) **EP 2 560 665 B1**
(45) Veröffentlichungstag und Bekanntmachung des Hinweises auf die Patenterteilung: **26.05.2021**
(21) Anmeldenummer: 11709432.6
(22) Anmeldetag: 23.03.2011
(51) Int. Cl.: A61K 36/575, A61K 31/01, A61K 8/06, A61K 8/34, A61K 8/46, A61K 8/97, A61K 8/9789, A61K 9/08, A61K 9/10, A61K 9/107, A61K 47/20, A61K 45/06, A61P 17/00, A61Q 19/00, A61Q 19/08

(54) **KOSMETISCHE ODER DERMATOLOGISCHE ZUBEREITUNGEN ENTHALTEND KOMBINATIONEN AUS MAGNOLIENRINDENEXTRAKT UND ALKYLSULFATEN**
COSMETICAL OR DERMATOLOGICAL COMPOSITIONS CONTAINING COMBINATIONS OF A MAGNOLIA BARK EXTRACT AND ALKYLSULPHATES
COMPOSITIONS COSMETIQUES OU DERMATOLOGIQUES COMPRENANT DES COMBINAISONS D'UN EXTRAIT D' ECORCE DE MAGNOLIA ET DES SULPHATES D'ALKYLE

(30) Priorität: 20.04.2010 DE 102010015789
(43) Veröffentlichungstag der Anmeldung: 27.02.2013
(73) Patentinhaber: Beiersdorf AG, 20253 Hamburg (DE)
(72) Erfinder: SCHLÄGER, Torsten, 22297 Hamburg (DE); ECKERT, Julia, 20249 Hamburg (DE); NEUFANG, Gitta, Hamburg 20149 (DE); PETERS, Nils, 22965 Todendorf (DE); WÖHRMANN, Michael, 22525 Hamburg (DE); HEUSER, Stefan, 90762 Fürth (DE)
(86) Internationale Anmeldenummer: PCT/EP2011/054396
(87) Internationale Veröffentlichungsnummer: WO 2011/131439

(56) Entgegenhaltungen:
- WO-A1-01/82922
- WO-A1-2004/065536
- WO-A1-2007/064519
- DE-A1-102007 028 508
- FR-A1- 2 911 507
- DE LA MAZA A ET AL: "Alkyl sulfate surfactants as solubilizing agents of liposomes modeling the composition of the stratum corneum lipids", COLLOIDS AND SURFACES A: PHYSICOCHEMICAL AND ENGINEERING ASPECTS 19990215 NL LNKD- DOI:10.1016/S0927-7757(98)00707-9, Bd. 147, Nr. 3, 15. Februar 1999 (1999-02-15), Seiten 341-348, XP002633461, ISSN: 0927-7757

## Beschreibung

Die vorliegende Erfindung betrifft kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Das Problem "sensible Haut" betrifft eine wachsende Anzahl Erwachsener und Kinder. Mit sensibler Haut bezeichnet man eine Kombination verschiedener Symptome, wie hyperreaktive und intolerante Haut. Aber auch atopische Haut kann darunter subsummiert werden. Diese Hautzustände werden von den Betroffenen oftmals, nicht ganz korrekt, als "allergische" Haut bezeichnet. Obwohl eine allergische Erkrankung zu Symptomen der sensiblen Haut führen kann, ist die Erscheinung "sensible Haut" nicht auf Allergiker beschränkt.

Die vorliegende Erfindung betrifft in einer besonderen Ausführungsform kosmetische Zubereitungen mit einem Gehalt an Magnolienrindenextrakt zur Steigerung der Mikrozirkulation der Haut sowie die Verwendung von Magnolienrindenextrakt zur Herstellung topischer Zubereitungen zur Steigerung der Mikrozirkulation der Haut.

Das Blutgefäßsystem dient der Versorgung von Geweben mit Nährstoffen, Vitaminen, Sauerstoff, dem Transport von Hormonen von Sekretions- zu Zielorganen und der Entsorgung von metabolischen Endprodukten wie CO₂ zum Ausscheidungsorgan Lunge oder Harnstoff zur Auscheidung via Niere.

Mit dem Begriff Mikrozirkulation bezeichnet man die Durchblutung und den Stoffaustausch in den kleinsten Blutgefäßen mit einer Größe kleiner als 100 µm (z. B. Kapillaren, Arteriolen, Venolen), den sogenannten Mikrogefäßen.

Die Mikrogefäße leiten das Blut und mit ihm lebensnotwendigen Sauerstoff und Nährstoffe zu jeder einzelnen Körperzelle und transportieren im Gegenzug Schlacken und Giftstoffe aus den Zellen ab. Im Kapillargebiet, der sogenannten Endstrombahn, fließt das Blut mit einer Geschwindigkeit von nur 1 cm in 20 s.

Dies ist erforderlich, um den in der Endstrombahn vorgesehenen Austausch der Nähr- und Abfallstoffe (Stoffwechselendprodukte) möglich zu machen. Um dieses langsame Fließen des Blutes zu ermöglichen und konstant zu halten, können sich vor allem die arteriellen Blutgefäße maximal erweitern oder verengen.

Eines der wenigen Gewebe, die physiologischerweise nicht durchblutet sind, ist die Epidermis. Sie wird über die Mikrokapillaren der Dermis, die in einem in der Jugend regelmäßigen Muster von Papillen (Retezapfen) in die Epidermis vorstoßen, versorgt. Diese Architektur wurde mittels der klassischen Histologie - Biposieentnahme, Fixierung, Färbung und zahllose Schnitte - ermittelt und erkannt. Die quantitative histometrische Beschreibung wird durch prozessbedingte Schrumpfung der Gewebeproben erschwert bzw. verzerrt. Trotz allem erlaubte die klassische Technik, altersbedingte Degeneration der Mikrokapillarsysteme und Einebnung der Retezapfen, d.h. der Grenzzone Epidermis / Dermis zu erkennen. Dies zieht eine verminderte Ver- und Entsorgung der Epidermis nach sich (Verkleinerung der Transportfläche). Auch das Ausmaß der Verzahnungsfläche Epidermis/Dermis wird geringer, was zu einer verminderten mechanischen Festigkeit und Elastizität der Haut führt.

Status und Veränderungen des Mikrokapillarsystems und des Retezapfenmusters lassen sich mittels moderner Techniken wie der konfokalen Mikroskopie in vivo - d.h. verletzungsfrei, beliebig oft wiederholbar und dynamisch auf aktuelle Einflüsse reagierend - an Probanden rasch (Minuten) darstellen, verfolgen und quantifizieren sowohl hinsichtlich der Dichte der Retezapfen bzw. Mikrokapillaren (Anzahl/Flächeneinheit) als auch in ihrer Höhe, was eine Abschätzung der Größe der Austauschfläche ermöglicht.

Regelmäßige topische Anwendung von Magnolienrindenextrakt auf der Haut älterer Probanden führt zu reversibel und im Bereich jugendlicher Haut erhöhter Retezapfen- und Mikrokapillarendichte. Diese strukturellen Veränderungen äußern sich sowohl in einer Verbesserung von Elastizitätsparametern der Haut (verlangsamte Bildung von Saugblasen) als auch des Feuchtigkeitsgehaltes und der Rauhigkeit der Hornschicht. Besonders ausgeprägt äußert sich diese juvenile Anatomie in der sie begleitenden optimierten Funktion bei der Antwort auf Stress (physikalischen Stress wie Kälte oder Trockenheit, agressive Agenzien wie Tenside, Stingings-Promotoren oder andere Irritantien, immunologisch in rascherer Abheilung von Dermatosen, Wundheilung oder bei mikrobiellem Befall usw.).

Magnolien (Magnolia) sind eine Pflanzengattung in der Familie der Magnoliengewächse (Magnoliaceae). Sie enthält etwa 230 Arten, die aus Ostasien und Amerika stammen.

Erfindungsgemäß kann der Magnolienrindenextrakt grundsätzlich allen Arten der Gattung Magnolia entnommen werden, wobei allerdings Magnolia grandiflora, und insbesondere Magnolia officinalis bevorzugt werden.

Magnolien-Rinde wird in der Traditionellen Chinesischen Medizin (TCM) vor allem bei "Stagnation of qi" (low energy), emotionalem Stress, Entzündungen, innerer Unruhe und Angstzuständen eingesetzt.

Die Herstellung des verwendeten Extraktes erfolgt durch Extraktion mit Hilfe von superkritischem CO₂ oder Ethanol oder einer Ethanol / Wasser Mischung.
Als typische Inhaltstoffe und Leitsubstanzen Leitsubstanzen des Extraktes sind Magnolol und Honokiol definiert.

Magnolol ist durch folgende Struktur gekennzeichnet:

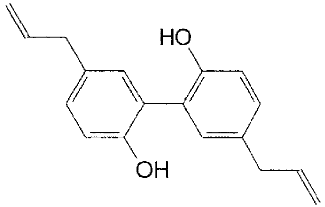

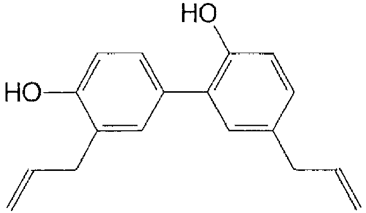

Honokiol ist durch folgende Struktur gekennzeichnet:
Nachteilig an Magnolienrindenextrakt ist seine geringe Löslichkeit in wäßrigen Zubereitungen, wodurch die Wirksamkeit messbar abnimmt.

Eine weitere Aufgabe der vorliegenden Erfindung war es daher, Zubereitungen zur Verfügung zu stellen, in welchen ein genügende Menge an Magnolienextrakt in gelöster Form vorliegt.

Es wurde überraschend gefunden, daß Natriumstearylsulfat die Löslichkeit von Magnolienrindenextrakt in wäßrigen Medien steigert.

Die Lösung dieser Aufgaben besteht erfindungsgemäß in kosmetischen oder dermatologischen Zubereitungen, enthaltend Kombinationen aus
a) Honokiol und Magnolol oder Magnolienrindenextrakt mit einem wirksamen Gehalt an Honokiol und Magnolol und
b) Natriumstearylsulfat.

Erfindungsgemäß ist schließlich die Verwendung von Natriumstearylsulfat zur Steigerung der Löslichkeit von Magnolienrindenextrakt in wäßrigen Medien.

Dabei sind Natriumstearylsulfat und Natriumcetylsulfat oder Mischungen aus beiden erfindungsgemäß bevorzugt.

Bevorzugt enthalten kosmetische oder dermatologische Zubereitungen gemäß der Erfindung 0,001 - 10 Gew.-%, besonders bevorzugt 0,01 - 1 Gew.-% Magnolienrindenextrakt, bezogen auf die Gesamtzusammensetzung der Zubereitungen.

Bevorzugt enthalten kosmetische oder dermatologische Zubereitungen gemäß der Erfindung 0,001 - 10 Gew.-% an Natriumstearylsulfat , bezogen auf das Gesamtgewicht der Zubereitungen.

Emulsionen sind vorteilhafte Darreichungsformen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

### Wirkungsnachweis

Es wurden jeweils 3 Ansätze mit sechs verschiedenen oberflächenaktiven Substanzen durchgeführt, bei denen jeweils die oberflächenaktive Substanz alleine, der Magnolienrindenextrakt alleine und eine Mischung aus oberflächenaktiver Substanz und Magnolienrindenextrakt in Wasser und Butylenglykol gelöst wurden.

Polyglyceryl-3-Methylglucose Distearat, Triceteareth-4-Phosphat, Glyceryl Stearat Citrat, PEG-40-Stearat und Na-Stearylsulfat verwendet.
Die Einsatzmengen sowie die Versuchsbedingungen waren bei allen Ansätzen identisch (2 g oberflächenaktive Substanz, 0,5g Magnolienrindenextrakt, 10 g Butylenglykol, Wasser ad 100g).

Nach zwei Monaten Lagerung wurden die Ansätze bewertet.

Bei Verwendung von Na-Stearylsulfat konnte, im Gegensatz zu den anderen getesteten Substanzen, überraschenderweise eine Verbesserung der Löslichkeit von Magnolia Bark Extrakt in wässrigen Lösungen gezeigt werden.

Die nachfolgenden Beispiele sollen die vorliegende Erfindung verdeutlichen.

### Rezepturbeispiele:

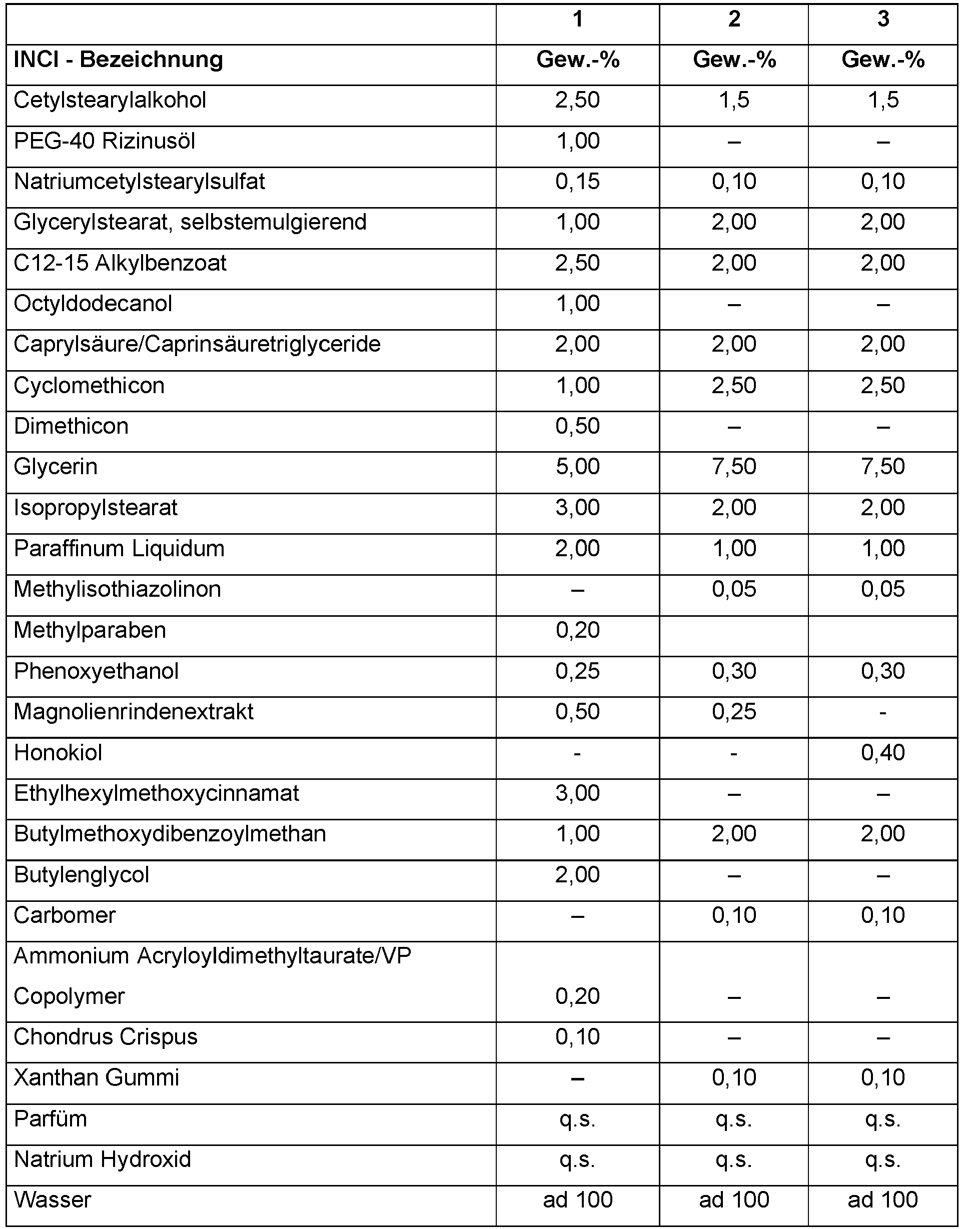

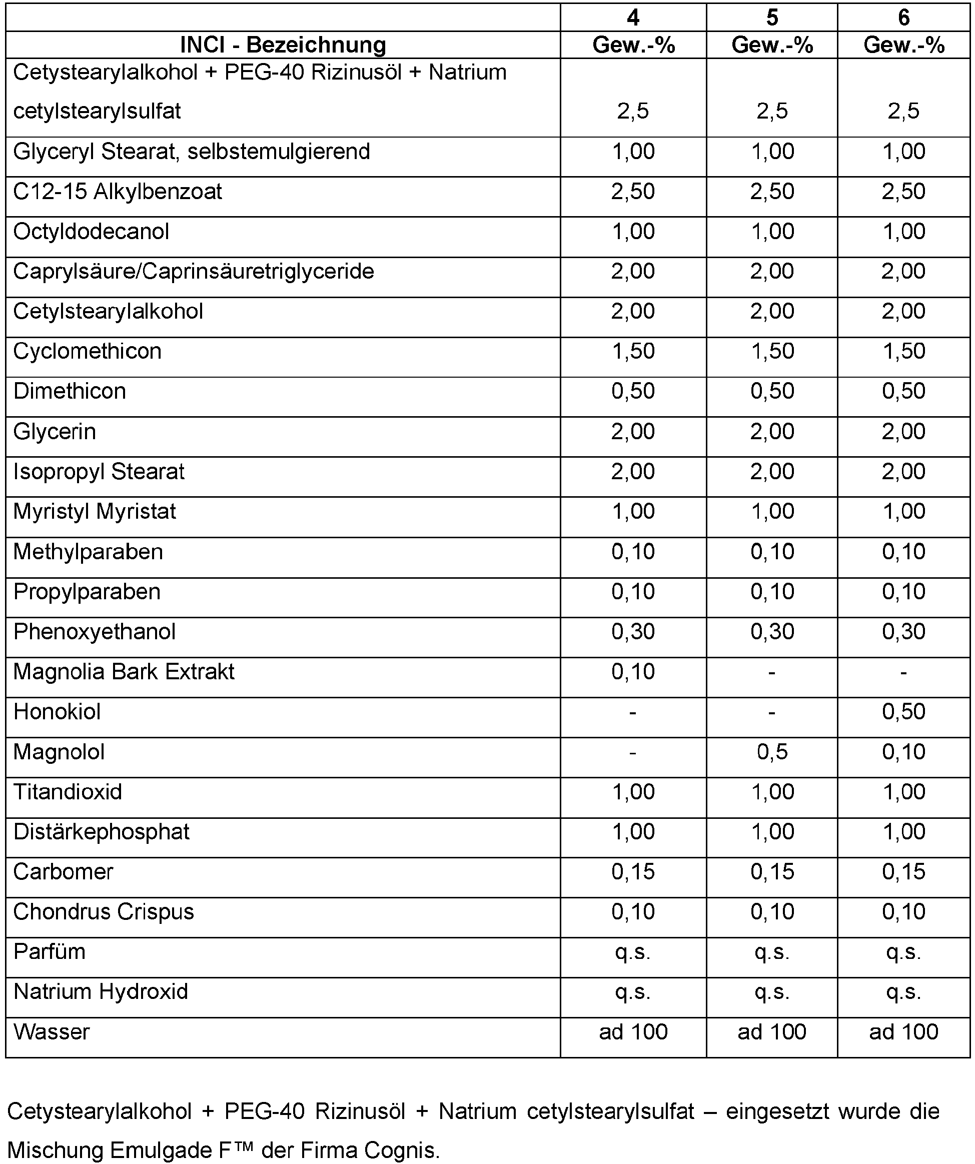

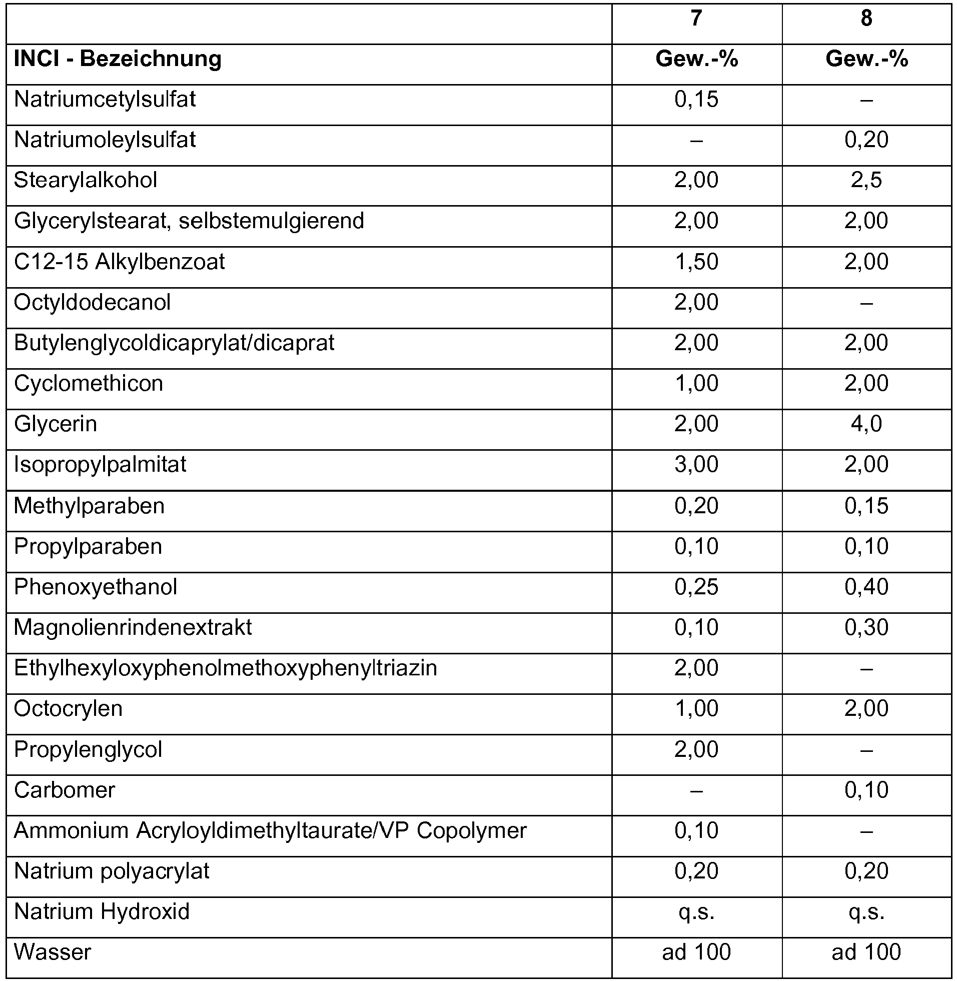

## Patentansprüche

1. Kosmetische oder dermatologische Zubereitungen in Emulsionsform, enthaltend Kombinationen aus
a) Honokiol und Magnolol oder Magnolienrindenextrakt mit einem wirksamen Gehalt an Honokiol und Magnolol und
b) Natriumstearylsulfat.

2. Verwendung von Natriumstearylsulfat zur Steigerung der Löslichkeit von Honokiol und Magnolol oder Magnolienrindenextrakt mit einem wirksamen Gehalt an Honokiol und Magnolol in wäßrigen Medien in Emulsionen.

3. Zubereitungen nach Anspruch 1 oder Verwendung nach Anspruch 2, wobei die Zubereitungen, 0,001 - 10 Gew.-% Magnolienrindenextrakt, bezogen auf das Gesamtgewicht der Zubereitungen enthalten.

4. Zubereitungen oder Verwendung nach einem der vorstehenden Ansprüche, wobei die Zubereitungen, 0,001 - 10 Gew.-% an Natriumstearylsulfat, bezogen auf das Gesamtgewicht der Zubereitungen enthalten.

5. Zubereitungen oder Verwendung nach einem der vorstehenden Ansprüche, wobei der Magnolienrindenextrakt durch Extraktion von Magnolienrinde
mit überkritischem CO₂ gewonnen wurde.

6. Zubereitungen nach einem der vorstehenden Ansprüche, **dadurch gekennzeichnet, daß** der Magnolienrindenextrakt mit einem Wasser / Ethanol-Gemisch als Extraktionsmittel gewonnen wird.

7. Zubereitungen oder Verwendung nach einem der vorstehenden Ansprüche, **dadurch gekennzeichnet, daß** der Magnolienrindenextrakt durch Extraktion von Rinde von Magnolia grandiflora, und/oder Magnolia officinalis gewonnen wird.

## Claims

1. Cosmetic or dermatological preparations in emulsion form, comprising combinations of
a) honokiol and magnolol or magnolia bark extract having an effective content of honokiol and magnolol and
b) sodium stearyl sulfate.

2. Use of sodium stearyl sulfate for increasing the solubility of honokiol and magnolol, or magnolia bark extract having an effective content of honokiol and magnolol, in aqueous media in emulsions.

3. Preparations according to Claim 1 or use according to Claim 2, wherein the preparations comprise 0.001 - 10% by weight magnolia bark extract, based on the total weight of the preparations.

4. Preparations or use according to any of the preceding claims, wherein the preparations comprise 0.001 - 10% by weight of sodium stearyl sulfate, based on the total weight of the preparations.

5. Preparations or use according to any of the preceding claims, wherein the magnolia bark extract has been obtained by extraction of magnolia bark with supercritical CO₂.

6. Preparations according to any of the preceding claims, **characterized in that** the magnolia bark extract is obtained using a water/ethanol mixture as extractant.

7. Preparations or use according to any of the preceding claims, **characterized in that** the magnolia bark extract is obtained by extraction of the bark of Magnolia grandiflora and/or Magnolia officinalis.

## Revendications

1. Préparations cosmétiques ou dermatologiques sous forme d'émulsion, contenant des combinaisons
a) d'honokiol et de magnolol ou d'extrait d'écorce de magnolias doté d'une teneur efficace en honokiol et en magnolol et
b) de stéarylsulfate de sodium.

2. Utilisation de stéarylsulfate de sodium pour l'augmentation de la solubilité d'honokiol et de magnolol ou d'extrait d'écorce de magnolias doté d'une teneur efficace en honokiol et en magnolol dans des milieux aqueux dans des émulsions.

3. Préparations selon la revendication 1 ou utilisation selon la revendication 2, les préparations contenant 0,001 à 10 % en poids d'extrait d'écorce de magnolias, par rapport au poids total des préparations.

4. Préparations ou utilisation selon l'une quelconque des revendications précédentes, les préparations contenant 0,001 à 10 % en poids de stéarylsulfate de sodium, par rapport au poids total des préparations.

5. Préparations ou utilisation selon l'une quelconque des revendications précédentes, l'extrait d'écorce de magnolias ayant été obtenu par extraction d'écorce de magnolias avec du CO₂ supercritique.

6. Préparations selon l'une quelconque des revendications précédentes, **caractérisées en ce que** l'extrait d'écorce de magnolias a été obtenu avec un mélange eau/éthanol en tant qu'agent d'extraction.

7. Préparations ou utilisation selon l'une quelconque des revendications précédentes, **caractérisée**(s) en ce que l'extrait d'écorce de magnolias a été obtenu par extraction d'écorce de Magnolia grandiflora et/ou Magnolia officinalis.
